# EUROPÄISCHE PATENTSCHRIFT

(11) **EP 2 021 041 B1**
(45) Veröffentlichungstag und Bekanntmachung des Hinweises auf die Patenterteilung: **23.12.2009**
(21) Anmeldenummer: 07725555.2
(22) Anmeldetag: 25.05.2007
(51) Int. Cl.: A61L 27/06

(54) **KNOCHEN-IMPLANTAT AUS EINER TITANLEGIERUNG SOWIE VERFAHREN ZU DEREN HERSTELLUNG**
BONE IMPLANT MADE OF A TITANIUM ALLOY, AND METHOD FOR THE PRODUCTION THEREOF
IMPLANT OSSEUX CONSTITUÉ D'UN ALLIAGE DE TITANE ET PROCÉDÉ DE RÉALISATION DUDIT ALLIAGE

(30) Priorität: 31.05.2006 DE 102006025292
(43) Veröffentlichungstag der Anmeldung: 11.02.2009
(73) Patentinhaber: GfE Metalle und Materialien GmbH, 90431 Nürnberg (DE)
(72) Erfinder: ACHTERMANN, Matthias, 90587 Veitsbronn (DE); BREME, Jürgen, 66265 Heusweiler/Saar (DE); GÜTHER, Volker, 90559 Burgthann (DE); KEZSA, Otto, 86399 Bobingen (DE); OTTO, Andreas, 90574 Rosstal (DE)
(74) Vertreter: Hübner, Gerd
(86) Internationale Anmeldenummer: PCT/EP2007/004659
(87) Internationale Veröffentlichungsnummer: WO 2007/137772

(56) Entgegenhaltungen:
- US-A- 4 857 269
- US-A- 5 871 595
- US-A1- 2002 162 608
- JAVAID I. QAZI AND HENRY J. RACK: "Metastable Beta Titanium Alloys for Orthopedic Applications" ADVANCED ENGINEERING MATERIALS, Bd. 7, Nr. 11, 2005, Seiten 993-998, XP002479976 in der Anmeldung erwähnt

## Beschreibung

Die Erfindung betrifft ein medizinisches Knochen-Implantat, welches zumindest teilweise aus einer Titanlegierung besteht sowie ein Verfahren zu deren Herstellung.

Zum Hintergrund der Erfindung ist festzuhalten, dass Titanlegierungen in der Medizintechnik auf unterschiedlichen Anwendungsgebieten wegen ihrer besonders guten Bioverträglichkeit eingesetzt werden. Ein Gebiet davon sind Knochen-Implantate, wie beispielsweise Hüft- oder Kniegelenk-Prothesen.

Bekannt gute Eigenschaften in diesem Zusammenhang zeigen Rein-Titan (TiCP) sowie die Titan-Legierungen TiA16V4 und TiA16Nb7. Diese Werkstoffe besitzen neben der erwähnten guten Körperverträglichkeit hohe Festigkeiten.

Problematisch bei Anwendung dieser Werkstoffe für Knochen-Implantate ist jedoch ihre Steifigkeit, da ihr Elastizitätsmodul bei ca. 100 GPa liegt und damit um einen Faktor 5 größer als das Elastizitätsmodul von Knochen (ca. 20 GPa) ist. Demzufolge entstehen unter Lasteinwirkung Relativbewegungen zwischen Implantat und Knochen, die zu einer Lockerung und damit zu einem Versagen des Implantates führen können. Dieses Problem ist besonders im Zusammenhang mit Re-Implantations-Prothesen bei Hüftgelenken und mit Dental-Implantaten gegeben. Insbesondere für diese Anwendungen besteht ein Bedürfnis für Werkstoffe mit einem niedrigeren Elastizitätsmodul.

In diesem Zusammenhang ist es aus dem Stand der Technik bekannt, das Elastizitätsmodul von Titan durch Zulegieren von Niob, Tantal, Molybdän, Zirkonium, Hafnium und/oder Vanadium zu senken. Dadurch sinkt jedoch grundsätzlich die Festigkeit der Legierung in Bereiche, die sie für den Einsatz als Implantatwerkstoff ungeeignet machen.

Die Festigkeitsabnahme kann zum Teil durch Wärmebehandlungen und/oder mechanische Umformungen verringert werden.

In der Fachliteratur, beispielsweise in dem Artikel "Metastable β-Titanium Alloys For Orthopedic Applications" in Advanced Engineering Materials 2005, 7, No. 11, Seiten 993 - 998 wird von Titanlegierungen mit Elastizitätsmoduli von 60 GPa bei Festigkeiten von ca. 1000 MPa berichtet.

Aus der umfangreichen Patentliteratur ist eine Vielzahl von teilweise kompliziert aufgebauten Titanlegierungen bekannt, deren Elastizitätsmoduli zwischen 40 und 50 GPa betragen sollen, die aber nicht oder nur in extrem geringem Umfang Eingang in die kommerzielle Verwertung gefunden haben. Als Gründe werden sehr oft Probleme mit enthaltenen toxischen Elementen aufgerührt, wobei die Toxizität von bestimmten Elementen wie beispielsweise Fe, Al oder Mo oft unterschiedlich bewertet v ird. Die US 5 871 595 beispielsweise offenbart eine biokompatible Titan-basierte Legierung, die zwischen 2 und 9 Atomprozent Zirkonium und etwa 22 bis 30 Atomprozent Niob plus Tantal enthält. Die Legierung soll frei von toxischen Metallelementen, wie beispielsweise Al, Ni, Co, Fe, Ca, Mo und W sein.

Die US 4 857 269 offenbart eine biokompatible Titan-basierte Legierung, die Niob- und Eisen-Bestandteile enthält. Ihr Elastizitätsmodul soll unter 100 GPa liegen, was für die Anwendung als Knochen-Implantat zu hoch liegt.

Aus der US 5 545 227 ist eine Hüftgelenksprothese bekannt, die aus einer Titan-Legierung mit Niob, Zirkon und Tantal besteht.

Auch die US 5 509 933 bezieht sich auf diesen Typ von Titan-Legierung für biokompatible medizinische Implantate mit hoher Festigkeit und geringem Elastizitätsmodul.

Die US 6 752 882 B2 schließlich offenbart eine biokompatible binäre Titan-Niob-Legierung, deren Niob-Anteil bei 10 bis 30 Gewichtsprozent, vorzugsweise 13 bis 28 Gewichtsprozent liegt. Diese Legierung wird als geeignet für orthopädische oder Dental-Implantate bezeichnet. Die in der Druckschrift berichteten Werte für das Elastizitätsmodul liegen bei über 60 GPa und sind damit für einen erfolgreichen Einsatz als Material für Knochen-Implantate immer noch erheblich verbesserungsbedürftig.

Ausgehend von der geschilderten Problematik des Standes der Technik liegt der Erfindung die Aufgabe zugrunde, ein medizinisches Knochenimplantat zu schaffen, welches zumindest teilweise aus einer biokompatiblen Titanlegierung mit hoher Festigkeit und niedrigem Elastizitätsmodul besteht.

Diese Aufgabe wird durch ein Implantat gemäß Anspruch 1 gelöst. Das erfindungsgemäße Implantat besteht zumindest teilweise aus einer Titanlegierung mit folgender Zusammensetzung:
- Niob mit einem Anteil zwischen 25,0 Masse-% und 30,0 Masse-%,
- Eisen und/oder Mangan mit einem Anteil zwischen 0,5 Masse-% und 3,0 Masse-%,
- Silizium mit einem Anteil zwischen 0,1 Masse-% und 1,0 Masse-%,
- Rest-Titan mit einem Anteil zwischen 66,0 Masse-% und 74,4 Masse-% einschließlich unvermeidbarer Verunreinigungen.

Versuche haben gezeigt, dass die vorstehende Legierungszusammensetzung grundsätzlich für einen Werkstoff geeignet ist, der eine Zugfestigkeit von mehr als 900 MPa und ein Elastizitätsmodul von kleiner als 50 GPa geeignet ist. Insgesamt ist das bevorzugte Verhältnis von Elastizitätsmodul zu Zugfestigkeit bei einem Wert von kleiner als 50.

Vorteilhafte Anteilsbereiche der Bestandteile liegen für Niob zwischen 27,5 Masse-% und 28,5 Masse-%, für Eisen und Mangan zwischen 0,8 Masse-% und 1,2 Masse-% sowie für Silizium zwischen 0,4 Masse-% und 0,6 Masse-%.

Insgesamt weist eine bevorzugte Titanlegierung eine Zusammensetzung von Titan zu 70,5 Masse-%, Niob zu 28,0 Masse-%, Eisen zu 1,0 Masse-% und Silizium zu 0,5 Masse-% auf.

Die Legierung ist dabei bevorzugt durch Kaltumformung in einen martensitischen Gefügezustand übergeführt.

Die Erfindung betrifft ferner ein Verfahren zur Herstellung einer wie vorstehend charakterisierten Titanlegierung, das folgende Verfahrensschritte aufweist:
- Erschmelzen eines Legierungsingots vorzugsweise durch Herstellung einer Vorlegierung aus Niob und Titan, sowie Zulegieren der Bestandteile Titan, Eisen und/oder Mangan sowie Silizium,
- Thermomechanische Behandlung der Legierung,
- Temperung der Legierung und
- Kaltumformung der Legierung in einen martensitischen Zustand.

Weitere Merkmale, Einzelheiten und Vorteile ergeben sich aus der nachfolgenden Beschreibung einer erfindungsgemäßen Legierung und des dafür eingesetzten Herstellungsverfahrens in einem

### Ausführungsbeispiel:

Die Legierungsbestandteile Titan mit einem Anteil von 30 Masse-% und Niob mit einem Anteil von 70 Masse-% (einschließlich unvermeidbarer Verunreinigungen) werden zu einer Vorlegierung geschmolzen. Das Schmelzaggregat ist ein Vakuum-Lichtbogen-Ofen (VAR) mit verzehrender Elektrode.

Anschließend werden die Vorlegierung mit einem Anteil von 40 Masse-%, Titan mit einem Anteil von 58,5 Masse-%, Eisen mit einem Anteil von 1 Masse-% und Silizium mit einem Anteil von 0,5 Masse-% im VAR zu einem zylindrischen Block (Ingot) erschmolzen. Zur Erzielung einer besseren Homogenität wird der Ingot noch mindestens einmal im VAR umgeschmolzen. Anschließend wird der Ingot aus der so gebildeten Ti -28Nb - 1Fe -0,5Si - Legierung mechanisch bearbeitet und bei einer Temperatur von 850° C stranggepresst, was zu einer Auflösung des Gussgefüges der Legierung führt. Eine anschließende Wärmebehandlung bei 900° C über 1 Stunde mit anschließender Wasserabschreckung dient zur Einstellung eines beta-Titan-Gefüges, um die weitere Umformbarkeit zu verbessern. Schließlich wird das Legierungsmaterial durch mehrere Kaltumformschritte bei Raumtemperatur mit Hilfe von Kaliberwalzen ohne Zwischenglühung in einen martensitischen Zustand überführt. Die martensitische Umwandlung wird ausschließlich durch Einbringung von mechanischer Umformenergie induziert. Dieses Material weist bei hoher Zugfestigkeit eine deutlich geringere Steifigkeit auf. Werte unter 40 GPa wurden im Rahmen von Versuchen bei der Entwicklung der vorliegenden Erfindung gemessen. Der Umformgrad bei der Kaltumformung liegt im Bereich zwischen 60 und 95 %.

Folgende mechanische Eigenschaften wurden für die vorstehend erörterte TI-28Nb-1Fe-0,5Si-Legierung ermittelt:
Streckgrenze Rp 0,2: 998NPa
Zugfestigkeit Rn: 1024MPa
Dehnung: 10,7 %
Elastizitätsmodul: 37,5 GPa.

## Patentansprüche

1. Medizinisches Knochen-Implantat, welches zumindest teilweise aus einer biokompatiblen Titanlegierung mit hoher Festigkeit und niedrigem Elastizitätsmodul besteht, wobei die Titanlegierung folgende Zusammensetzung aufweist:
- Niob mit einem Anteil zwischen 25,0 Masse-% und 30,0 Masse-%,
- Eisen und/oder Mangan mit einem Anteil zwischen 0,5 Masse-% und 3,0 Masse-%,
- Silizium mit einem Anteil zwischen 0,1 Masse-% und 1,0 Masse-%,
- Rest mindestens 66,0 Masse-% Titan einschließlich unvermeidbarer Verunreinigungen.

2. Medizinisches Knochen-Implantat nach Anspruch 1, **gekennzeichnet durch** einen Niob-Anteil der Titanlegierung zwischen 27,5 Masse-% und 28,5 Masse-%.

3. Medizinisches Knochen-Implantat nach Anspruch 1 oder 2, **gekennzeichnet durch** einen Eisen-Anteil der Titanlegierung zwischen 0,8 Masse-% und 1,2 Masse-% und einen Mangangehalt von 0 %.

4. Medizinisches Knochen-Implantat nach Anspruch 1 oder 2, **gekennzeichnet durch** einen Mangan-Anteil der Titanlegierung zwischen 0,8 Masse-% und 1,2 Masse-% und einen Eisenanteil von 0 %.

5. Medizinisches Knochen-Implantat nach Anspruch 1, 2 oder 3, **gekennzeichnet durch** einen Silizium-Anteil der Titanlegierung zwischen 0,4 Masse-% und 0,6 Masse-%.

6. Medizinisches Knochen-Implantat nach einem der vorgenannten Ansprüche, **gekennzeichnet durch** folgende Anteile der Titanlegierung:
- Titan zu 70,5 Masse-%,
- Niob zu 28,0 Masse-%,
- Eisen zu 1,0 Masse-% und
- Silizium zu 0,5 Masse-%.

7. Medizinisches Knochen-Implantat nach einem der vorgenannten Ansprüche, **dadurch gekennzeichnet, dass** die Legierung durch Kaltumformung in einen martensitischen Gefügezustand übergeführt ist.

8. Medizinisches Knochen-Implantat nach einem der vorgenannten Ansprüche, **dadurch gekennzeichnet, dass** die Zugfestigkeit der Legierung einen Wert von größer als 900 MPa aufweist.

9. Medizinisches Knochen-Implantat nach einem der vorgenannten Ansprüche, **dadurch gekennzeichnet, dass** das Elastizitätsmodul der Legierung einen Wert von kleiner als 50 GPa, vorzugsweise von kleiner als 40 GPa aufweist.

10. Medizinisches Knochen-Implantat nach Anspruch 8 oder 9, **dadurch gekennzeichnet, dass** das Verhältnis von Elastizitätsmodul zu Zugfestigkeit einen Wert von kleiner als 50 aufweist.

11. Verfahren zur Herstellung einer Titanlegierung mit einer Zusammensetzung gemäß einem der Ansprüche 1 bis 6, **gekennzeichnet durch** folgende Verfahrensschritte:
- Erschmelzen eines Legierungsingots,
- Thermomechanische Behandlung der Legierung,
- Wärmebehandlung der thermomechanisch behandelten Legierung und
- Kaltumformung der Legierung in einen martensitischen Gefügezustand,
**dadurch gekennzeichnet, dass** der Legierungsingot **durch** Herstellen einer Vorlegierung aus Titan und Niob und anschließendes Zulegieren von Titan, Eisen und/oder Mangan und Silizium hergestellt wird.

12. Verfahren nach Anspruch 11, **dadurch gekennzeichnet, dass** die thermomechanische Behandlung durch ein Strangpressen bei vorzugsweise 850°C erfolgt.

13. Verfahren nach Anspruch 11 oder 12, **dadurch gekennzeichnet, dass** die Wärmebehandlung bei einer Temperatur von über 800°C, vorzugsweise bei 900°C über eine Dauer von 1 Stunde erfolgt.

14. Verfahren nach Anspruch 13, **dadurch gekennzeichnet, dass** die wärmebehandelte Legierung abgeschreckt wird.

15. Verfahren nach einem der Ansprüche 11 bis 14, **dadurch gekennzeichnet, dass** die Kaltumformung durch Walzen mit einem Umformgrad von vorzugsweise 60% bis 95% erfolgt.

## Claims

1. Medical bone implant, which consists at least partly of a biocompatible titanium alloy with high strength and a low module of elasticity, wherein the titanium alloy has the following composition:
- niobium in a proportion of between 25.0 mass % and 30.0 mass %,
- iron and/or manganese in a proportion of between 0.5 mass % and 3.0 mass %,
- silicon in a proportion of between 0.1 mass % and 1.0 mass %,
- remainder of at least 66.0 mass % titanium including unavoidable impurities.

2. Medical bone implant according to claim 1, **characterised by** a proportion of niobium in the titanium alloy of between 27.5 mass % and 28.5 mass %.

3. Medical bone implant according to claim 1 or 2, **characterised by** a proportion of iron in the titanium alloy of between 0.8 mass % and 1.2 mass % and a manganese content of 0%.

4. Medical bone implant according to claim 1 or 2, **characterised by** a proportion of manganese in the titanium alloy of between 0.8 mass % and 1.2 mass % and an iron content of 0 %.

5. Medical bone implant according to claim 1, 2 or 3, **characterised by** a proportion of silicon in the titanium alloy of between 0.4 mass % and 0.6 mass %.

6. Medical bone implant according to one of the preceding claims, **characterised by** the following proportions of titanium alloy:
- titanium - 70.5 mass %,
- niobium - 28.0 mass %,
- iron - 1.0 mass % and
- silicon - 0.5 mass %.

7. Medical bone implant according to one of the preceding claims, **characterised in that** the alloy is brought by cold forming into a martensitic structural state.

8. Medical bone implant according to one of the preceding claims, **characterised in that** the tensile strength of the alloy has a value of more than 900 MPa.

9. Medical bone implant according to one of the preceding claims, **characterised in that** the module of elasticity of the alloy has a value of less than 50 GPa, preferably less than 40 GPa.

10. Medical bone implant according to claim 8 or 9, **characterised in that** the ratio of the module of elasticity to the tensile strength has a value of less than 50.

11. Method for producing a titanium alloy with a composition according to one of claims 1 to 6, **characterised by** the following method steps:
- smelting an alloy ingot,
- thermomechanically treating the alloy,
- heat treating the thermomechanically treated alloy and
- cold forming the alloy into a martensitic structural state,
**characterised in that** the alloy ingot is produced by producing a master alloy of titanium and niobium and the subsequent additional alloying of titanium, iron and/or manganese and silicon.

12. Method according to claim 11, **characterised in that** the thermomechanical treatment is performed by extrusion moulding at preferably 850°C.

13. Method according to claim 11 or 12, **characterised in that** the heat treatment is conducted at a temperature greater than 800°C, preferably at 900°C, over a period of 1 hour.

14. Method according to claim 13, **characterised in that** the heat-treated alloy is quenched.

15. Method according to one of claims 11 to 14, **characterised in that** the cold forming is performed by rolling with a degree of forming of preferably 60% to 95%.

## Revendications

1. Implant osseux médical constitué au moins en partie d'un alliage de titane biocompatible présentant une résistance élevée et un faible module d'élasticité, l'alliage de titane comprenant la composition suivante :
- du Niob avec un pourcentage compris entre 25,0 % en masse et 30,0 % en masse,
- du fer et/ou du manganèse avec un pourcentage compris entre 0,5 % en masse et 3,0 % en masse,
- du silicium avec un pourcentage compris entre 0,1 % en masse et 1,0 % en masse,
- le reste étant au moins 66,0 % en masse de titane y compris les impuretés inévitables.

2. Implant osseux médical selon la revendication 1, **caractérisé par** un pourcentage de Niob de l'alliage de titane compris entre 27,5 % en masse et 28,5 % en masse.

3. Implant osseux médical selon la revendication 1 ou 2, **caractérisé par** un pourcentage de fer de l'alliage de titane compris entre 0,8 % en masse et 1,2 % en masse et une teneur en manganèse de 0 %.

4. Implant osseux médical selon la revendication 1 ou 2, **caractérisé par** un pourcentage de manganèse de l'alliage de titane compris entre 0,8 % en masse et 1,2 % en masse et un pourcentage de fer de 0 %.

5. Implant osseux médical selon la revendication 1, 2 ou 3, **caractérisé par** un pourcentage de silicium de l'alliage de titane compris entre 0,4 % en masse et 0,6 % en masse.

6. Implant osseux médical selon l'une quelconque des revendications précédentes, **caractérisé par** les pourcentages suivants de l'alliage de titane :
- titane pour 70,5 % en masse,
- Niob pour 28,0 % en masse,
- fer pour 1,0 % en masse et
- silicium pour 0,5 % en masse.

7. Implant osseux médical selon l'une quelconque des revendications précédentes, **caractérisé en ce que** l'alliage est amené à passer dans un état de structure de référence martensitique par formage à froid.

8. Implant osseux médical selon l'une quelconque des revendications précédentes, **caractérisé en ce que** la résistance à la traction de l'alliage comprend une valeur supérieure à 900 MPa.

9. Implant osseux médical selon l'une quelconque des revendications précédentes, **caractérisé en ce que** le module d'élasticité de l'alliage comprend une valeur inférieure à 50 GPa, de préférence inférieure à 40 GPa.

10. Implant osseux médical selon la revendication 8 ou 9, **caractérisé en ce que** le rapport entre le module d'élasticité et la résistance à la traction comprend une valeur inférieure à 50.

11. Procédé de fabrication d'un alliage de titane comprenant une composition selon l'une des revendications 1 à 6, **caractérisé par** les étapes de procédé suivantes consistant à :
- faire fondre un lingot d'alliage,
- effectuer un traitement thermomécanique de l'alliage,
- effectuer un traitement thermique de l'alliage traité de façon thermomécanique et
- former à froid l'alliage dans un état de structure de référence martensitique,
**caractérisé en ce que** le lingot d'alliage est fabriqué par fabrication d'un alliage-maître composé de titane et de Niob puis ajout de titane, fer et/ou manganèse et silicium.

12. Procédé selon la revendication 11, **caractérisé en ce que** le traitement thermomécanique s'effectue par extrusion à 850°C de préférence.

13. Procédé selon la revendication 11 ou 12, **caractérisé en ce que** le traitement thermique s'effectue à une température supérieure à 800°C, de préférence à 900°C pendant une durée de 1 heure.

14. Procédé selon la revendication 13, **caractérisé en ce que** l'alliage ayant subi un traitement thermique est trempé.

15. Procédé selon l'une quelconque des revendications 11 à 14, **caractérisé en ce que** la formation à froid s'effectue par des cylindres à un degré de formage de préférence de 60 % à 95 %.
